# EUROPÄISCHE PATENTSCHRIFT

(11) **EP 2 359 743 B1**
(45) Veröffentlichungstag und Bekanntmachung des Hinweises auf die Patenterteilung: **05.12.2012**
(21) Anmeldenummer: 11166277.1
(22) Anmeldetag: 24.07.2007
(51) Int. Cl.: A61B 3/10, A61B 3/12, A61B 3/00

(54) **Ophthalmologische Messvorrichtung und Messverfahren**
Ophthalmologic measuring device and measuring method
Dispositif de mesure ophtalmologique et procédé de mesure

(43) Veröffentlichungstag der Anmeldung: 24.08.2011
(62) Teilanmeldung aus: 07405214.3
(73) Patentinhaber: SIS AG, Surgical Instrument Systems, 2562 Port (CH)
(72) Erfinder: Rathjen, Christian, 28197, Bremen (DE)
(74) Vertreter: Rentsch Partner AG

(56) Entgegenhaltungen:
- EP-A1- 1 430 829
- WO-A-02/17775
- WO-A-2005/074789
- DE-A1- 19 713 138
- US-A- 5 347 328
- US-A- 5 387 951
- US-A1- 2005 203 422
- US-B1- 6 454 761
- US-B1- 6 779 891

## Beschreibung

### Technisches Gebiet

Die vorliegende Erfindung betrifft eine ophthalmologische Messvorrichtung und ein ophthalmologisches Messverfahren zum Bestimmen der axialen Länge eines Auges. Die vorliegende Erfindung betrifft insbesondere eine ophthalmologische Messvorrichtung und ein ophthalmologisches Messverfahren zur berührungsfreien Bestimmung der axialen Länge des Auges.

### Stand der Technik

Wie beispielsweise in US 5,347,328 erläutert wird, weisen herkömmliche Vorrichtungen zum interferometrischen Messen der axialen Augenlänge, in denen Laserstrahlen mit parallelem Strahlenverlauf auf der Retina konvergierend auf das Auge gestrahlt werden, den Nachteil auf, dass das von der Retina reflektierte Licht ausserhalb des Auges mit parallelem Strahlengang empfangbar ist, wohingegen das von der Cornea reflektierte Licht durch die Cornea divergent reflektiert wird, so dass für das von der Retina und das von der Cornea reflektierte Licht unterschiedliche Wellenfrontverläufe resultieren. Auf Grund der unterschiedlichen Wellenfrontverläufe ergeben sich bei der Detektierung von Interferenzstreifen mit kleinflächigem Lichtempfänger nur schwache Signale, die sich kaum vom durch die raue Retinaoberfläche verursachten Rauschen unterscheiden lassen, und bei der Detektierung mit grossflächigem Lichtempfänger ergeben sich mehrere Interferenzstreifen, die die Signalmodulation reduzieren und damit auch keine eindeutigen Rückschlüsse auf die Distanz zwischen Cornea und Retina zulassen. Zur Überwindung dieser Probleme wird nach US 5,347,328 das von der Cornea und das von der Retina reflektierte Licht mittels eines Strahlteilers verschiedenen optischen Lichterfassungssystemen zugeführt. In einem optischen Interferenzlichtempfänger wird das in den verschiedenen Lichterfassungssystemen empfangene Licht zusammengeführt und die Interferenz bestimmt. Gemäss US 5,347,328 sind somit für die Erfassung des an der Cornea und des and der Retina reflektierten Lichts und die darauf basierte interferometrische Bestimmung der axialen Augenlänge zwei unterschiedlich dimensionierte optische Beleuchtungs- und Erfassungssysteme erforderlich. Weiterhin ist es erforderlich zur Vermeidung parasitärer Interferenzen eine Weglängenkompensierung zu verwenden, die die Erfassungssysteme auf genau die gleiche optische Weglänge einstellt. Allgemein kann gesagt werden, dass die simultane Erfassung von Retina und Cornea einen im Vergleich zum einfachen Michelson-Interferometer doppelt so hohen gerätetechnischen Aufwand erfordert. Eine simultane Erfassung ist zur Vermeidung von Messfehlern durch axiale Augenbewegungen während des Messvorganges erforderlich. US 5,347,328 wie auch US 6,779,891 benutzen dazu interferometrische Aufbauten, die nur den relativen Abstand von optischen Grenzflächen, nicht aber den Abstand zum Messgerät, bestimmen können. Bei mehr als zwei optischen Grenzflächen kann deren Lage zueinander nicht eindeutig bestimmt werden. Würden solche Messverfahren zur Bestimmung weiterer intraokularer Distanzen eingesetzt, so liesse sich beispielsweise nicht zwischen der Vorderkammertiefe und der Linsendicke unterscheiden.

US 5,387,951 beschreibt eine ophthalmologische Messvorrichtung zum Bestimmen der axialen Länge eines Auges basierend auf einer interferometrischen Bestimmung einer Relativposition der Retina des Auges, und einer nicht-interferometrischen Bestimmung einer Relativposition der Cornea des Auges. Die Messvorrichtung gemäss US 5,387,951 ist eingerichtet für die nicht-interferometrische Bestimmung der Relativposition der Cornea Licht ringförmig auf die Cornea zu projizieren, das durch die Cornea reflektierte Licht als ringförmiges Bild zu erfassen, und die Position des Scheitelpunkts der Cornea basierend auf dem ringförmigen Bild zu bestimmen.

### Darstellung der Erfindung

Es ist eine Aufgabe der vorliegenden Erfindung eine ophthalmologische Messvorrichtung und ein ophthalmologisches Messverfahren zum Bestimmen der axialen Länge eines Auges vorzuschlagen, welche mindestens gewisse Nachteile des Stands der Technik nicht aufweisen, insbesondere sollen nicht unterschiedlich dimensionierte optische Erfassungssysteme für die Bestimmung von Interferenzmustern von an der Retina und der Cornea reflektiertem Licht erforderlich sein.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass die ophthalmologische Messvorrichtung zum Bestimmen der axialen Länge eines Auges, ein optisches interferometrisches erstes Messsystem zur Bestimmung einer Relativposition der Retina des Auges umfasst, dass die ophthalmologische Messvorrichtung zudem ein nicht-interferometrisches zweites Messsystem zur Bestimmung einer Relativposition der Cornea des Auges umfasst, und dass die ophthalmologische Messvorrichtung Verarbeitungsmittel zum Bestimmen der axialen Länge des Auges basierend auf der durch das interferometrische Messsystem bestimmten Relativposition der Retina und der durch das nicht-interferometrische Messsystem bestimmten Relativposition der Cornea umfasst. Das interferometrische Messsystem umfasst vorzugsweise eine Vorrichtung zur optischen Kohärenztomografie mit Tiefenabtastung. Mit dem Begriff "Relativposition" ist eine definierte geometrische Lage gemeint, beispielsweise mit Bezug zur ophthalmologischen Vorrichtung und beispielsweise auf einer definierten Messachse. Durch die Ausführung der ophthalmologischen Messvorrichtung mit einem interferometrischen und einem nicht-interferometrischen Messsystem für jeweils einen unterschiedlichen Messbereich auf einer Messachse, insbesondere einen ersten Messbereich im Corneabereich und einen zweiten Messbereich im Retinabereich, wird eine im Wesentlichen zeitgleiche Bestimmung der Relativpositionen sowohl der Cornea als auch der Retina ermöglicht, ohne dass dafür unterschiedlich dimensionierte optische Erfassungssysteme für die Bestimmung von Interferenzmustern von an der Retina und der Cornea reflektiertem Licht erforderlich sind. Es besteht dadurch zum einen der Vorteil, dass das interferometrische System ganz auf die Signalgüte der Retinamessung optimiert werden kann. Zum anderen gibt es keine Störungen, die durch ein anderes interferometrisches System verursacht werden können. Somit wird eine gegenüber dem Stand der Technik gerätetechnisch wesentlich einfachere Bestimmung der axialen Augenlänge ermöglicht, welche auf Grund der zeitgleichen Erfassung von Retina und Cornea nicht durch Augenbewegungen beeinträchtigt wird. Die Kombination von interferometrischer und nicht-interferometrischer Messung ermöglicht zudem nicht bloss die eindeutige Bestimmung der axialen Augenlänge, sondern mit der nicht-interferometrisch bestimmten relativen Corneaposition als eindeutiger Referenzpunkt in entsprechenden Ausführungsvarianten überdies auch die Bestimmung der Vorderkammertiefe (von der äusseren Corneaoberfläche bis zur Linse des Auges) und der Linsendicke. Weiterhin bestehen durch die Ermittlung einer Relativposition zum Messgerät, weitere Vorteile. Zum Beispiel kann mit Hilfe dieser Information ohne zusätzliche Hilfsmittel das Positionieren des Patienten erleichtert werden. Überdies ergeben sich durch die Kombination von interferometrischem und nichtinterferometrischem Messsystem Kostenvorteile gegenüber der gerätetechnisch aufwendigeren Ausgestaltung von bekannten Systemen, die die Retina und Cornea mittels interferometrischer Messtechnik simultan erfassen.

Für die Bestimmung der Vorderkammertiefe ist das interferometrische Messsystem oder das nicht-interferometrische Messsystem überdies eingerichtet, die Relativposition der der Cornea zugewandten Vorderseite der Linse des Auges zu bestimmen, und die Verarbeitungsmittel sind zudem eingerichtet, die Vorderkammertiefe des Auges basierend auf der Relativposition der Cornea und der Relativposition der zugewandten Vorderseite der Linse zu bestimmen. Für die Bestimmung der Linsendicke ist das interferometrische Messsystem oder das nicht-interferometrische Messsystem überdies eingerichtet, die Relativposition der der Cornea zugewandten Vorderseite der Linse zu bestimmen, und das interferometrische Messsystem ist zudem eingerichtet, die Relativposition der von der Cornea abgewandten Rückseite der Linse zu bestimmen, und die Verarbeitungsmittel sind zudem eingerichtet, die Dicke der Linse basierend auf der Relativpositionen der zugewandten Vorderseite und der Relativpositionen der abgewandten Rückseite zu bestimmen.

In einer Ausführungsvariante umfasst das nicht-interferometrische Messsystem ein Lichtschrankensystem zur Detektierung des Scheitelpunkts der Cornea als Relativposition der Cornea. Das Lichtschrankensystem ist so mit dem ersten Messsystem gekoppelt, dass die Detektierung des Scheitelpunkts automatisch das interferometrische Messsystem zur Bestimmung der Relativposition der Retina aktiviert. Insbesondere bei der Verwendung optischer Kohärenztomografie (OCT, Optical Coherence Tomographie) ohne bewegte optische Teile für die Tiefenabtastung (wird auch als statische Tiefenabtastung bezeichnet), also ohne veränderliche Referenzarmlänge, ermöglicht die Auslösung der interferometrischen Messung der Retina durch die Lichtschrankendetektierung der Cornea eine nur geringfügig verzögerte, im Wesentlichen zeitgleiche Bestimmung der Relativpositionen von Cornea und Retina und damit eine durch Augenbewegungen nicht beeinträchtige Bestimmung der axialen Augenlänge.

In verschiedenen alternativen Ausführungsvarianten umfasst das nicht-interferometrische Messsystem ein Abstandsmesssystem zur Bestimmung der Relativposition der Cornea auf der Basis von vom Auge diffus reflektiertem und/oder gestreutem Licht, oder auf der Basis von von der Cornea spiegelnd reflektiertem Licht.

Vorzugsweise umfasst das nicht-interferometrische Messsystem einen Lichtprojektor zur Projektion mindestens eines Lichtstrahls auf die Cornea, und einen mit den Verarbeitungsmitteln verbundenen Lichtempfänger. Die Verarbeitungsmittel sind zudem eingerichtet, die Relativposition der Cornea auf Grund eines vom Lichtempfänger empfangenen und erfassten Signals zu bestimmen. In verschiedenen Ausführungsvarianten sind der Lichtprojektor und der Lichtempfänger so kombiniert und angeordnet, dass die Relativposition der Cornea durch Lichtschrankendetektierung, oder durch Abstandsmessung auf der Basis diffuser Reflexion/Streuung oder spiegelnder Reflexion bestimmt wird.

In einer Ausführungsvariante ist der Lichtprojektor eingerichtet, zwei sich in einem Kreuzungspunkt schneidende Lichtstrahlen zu projizieren, und der Lichtempfänger ist eingerichtet, zur Bestimmung der Relativposition der Cornea die Positionierung des Kreuzungspunkts auf der Cornea zu detektieren. Neben dem Einsatz für die Abstandsmessung kann die Detektierung des Kreuzungspunkts auf der Cornea kann auch für die Lichtschrankendetektierung der Cornea eingesetzt werden.

In einer weiteren Ausführungsvariante umfasst das nicht-interferometrische Messsystem ein optisches System, z.B. eine Linse, zur fokussierten Projektion des Lichtstrahls auf die Cornea, und eine dem Lichtempfänger vorgeschaltete Lochblende. Dabei sind das optische System und die Lochblende so angeordnet, dass zur Bestimmung der Relativposition der Cornea der von der Cornea reflektierte Lichtstrahl über das optische System und durch die Lochblende dem Lichtempfänger zugeführt wird. Zum Beispiel wird bei einer Positionierung einer optischen Linse in der Mitte zwischen Cornea und Lochblende, dem Lichtempfänger zur Bestimmung der Relativposition der Cornea der von der Cornea reflektierte Lichtstrahl fokussiert durch die Lochblende zugeführt wird. Auch die Detektierung einer definierten Lage der Cornea zur Lochblende kann neben dem Einsatz für die Abstandsmessung auch für die Lichtschrankendetektierung der Cornea eingesetzt werden.

In einer Ausführungsvariante umfasst das Abstandsmesssystem einen Autofokussensor zur Bestimmung der Relativposition der Cornea. Der Autofokussensor basiert beispielsweise auf einer Lichtschrankendetektierung mit automatischer Verschiebung der gesamten Lichtschranke, der oben beschriebenen Lochblende oder der Lichtprojektoren für die sich kreuzenden Lichtstrahlen.

In einer weiteren Ausführungsvariante umfasst das Abstandsmesssystem einen Sensor zur Detektierung von wellenlängenabhängigen Brennpunkten bei Fokussierung einer breitbandigen Lichtquelle mit einer Optik mit starken chromatischen Aberrationen, und die Verarbeitungsmittel sind eingerichtet, auf Grund der chromatischen Aberration die Relativposition der Cornea zu bestimmen.

In einer anderen Ausführungsvariante ist der Lichtprojektor eingerichtet, den Lichtstrahl entlang einer Messachse durch einen Querschnittteil der Cornea zu projizieren, und der Lichtempfänger ist eingerichtet, ein Querschnittabbild des durch den Lichtprojektor beleuchteten Querschnittteils in Scheimpfluganordnung zum Lichtstrahl zu erfassen und zu speichern. Die Verarbeitungsmittel sind eingerichtet, die Relativposition der Cornea auf der Messachse basierend auf dem Querschnittabbild und der durch das interferometrische Messsystem auf der Messachse bestimmten Relativposition der Retina zu bestimmen.

In einer weiteren Ausführungsvariante ist das erste Messsystem so mit dem zweiten Messsystem gekoppelt, direkt oder über ein Steuermodul, dass ein die Detektierung der Relativposition der Retina anzeigendes Signal vom ersten Messsystem automatisch das zweite Messsystem zur Bestimmung der Relativposition der Cornea aktiviert. Insbesondere bei der Verwendung optischer Kohärenztomografie mit einer zur Bestimmung der Relativposition der Retina veränderbaren Referenzarmlänge (wird auch als dynamische Tiefenabtastung bezeichnet), ermöglicht die Auslösung der nicht-interferometrischen Messung der Cornea durch die interferometrische Bestimmung der Relativposition der Retina eine nur geringfügig verzögerte, im Wesentlichen zeitgleiche Bestimmung der Relativpositionen von Cornea und Retina und damit eine durch Augenbewegungen nicht beeinträchtige Bestimmung der axialen Augenlänge. Neben der Veränderung der Referenzarmlänge ist es auch möglich das gesamte Interferometer zu verschieben.

In einer alternativen Ausführungsvariante, sind das interferometrische Messsystem und das nicht-interferometrische Messsystem mit den Verarbeitungsmitteln verbunden und eingerichtet, laufend Signale zur Bestimmung der aktuellen Relativposition der Retina und zur Bestimmung der aktuellen Relativposition der Cornea an die Verarbeitungsmittel zu übertragen. Die Verarbeitungsmittel sind entsprechend eingerichtet, einen aktuell ermittelten Wert der axialen Länge des Auges basierend auf der aktuellen Relativposition der Retina und der aktuellen Relativposition der Cornea zu bestimmen.

In einer weiteren Ausführungsvariante umfassen das interferometrische Messsystem und das nicht-interferometrische Messsystem jeweils eine Lichtquelle zur Erzeugung eines Lichtstrahls, wobei die Wellenlängen der von den Lichtquellen erzeugten Lichtstrahlen voneinander verschieden sind. Die Erzeugung und Verwendung von Lichtstrahlen mit unterschiedlichen Wellenlängen ermöglicht die im Wesentlichen gleichzeitige Bestimmung der Relativpositionen von Retina und Cornea ohne gegenseitige Beeinflussung und Störung des interferometrischen und nicht-interferometrischen Messsystems. An dieser Stelle soll angefügt werden, dass in einer alternativen Ausführungsvariante für die beiden Messsysteme eine gemeinsame Lichtquelle oder zwei Lichtquellen mit gleicher Wellenlänge verwendet werden, wobei das nicht-interferometrische System so ausgelegt ist, dass es nicht vom interferometrischen Messsystem gestört wird (z.B. über Feldblenden oder konfokale Abbildungen).

Neben der ophthalmologischen Messvorrichtung zum Bestimmen der axialen Länge eines Auges betrifft die vorliegende Erfindung zudem ein Messverfahren zur berührungsfreien Bestimmung der axialen Länge eines Auges, wobei eine Relativposition der Retina des Auges mittels eines optischen interferometrischen Messsystems bestimmt wird, wobei eine Relativposition der Cornea des Auges mittels eines nicht-interferometrischen Messsystem bestimmt wird, und wobei die axiale Länge des Auges basierend auf der interferometrisch bestimmten Relativposition der Retina und der nicht-interferometrisch bestimmten Relativposition der Cornea berechnet wird. In verschiedenen Ausführungsvarianten wird mittels eines Lichtschrankensystems ein Scheitelpunkt der Cornea als Relativposition der Cornea detektiert und bei der Detektierung des Scheitelpunkts automatisch das optische interferometrische Messsystem zur Bestimmung der Relativposition der Retina aktiviert, oder die Relativposition der Retina wird mittels einer Vorrichtung zur optischen Kohärenztomografie z. B. durch Verändern einer Referenzarmlänge bestimmt und beim Detektieren eines die Relativposition der Retina anzeigenden Signals wird automatisch die Relativposition der Cornea mittels des nicht-interferometrischen Messsystems z. B. in der aktuellen Referenzarmlänge (z. B. der aktuellen Position des Referenzarmspiegels) bestimmt. Es besteht zudem auch die Möglichkeit, dass mittels des interferometrischen Messsystems und des nicht-interferometrischen Messsystems laufend aktuelle Relativpositionen der Retina und der Cornea bestimmt werden und darauf basierend ein aktueller Wert der axialen Länge des Auges berechnet wird. Zum Kalibrieren der ophthalmologischen Messvorrichtung wird vor der Bestimmung der Relativposition der Retina und der Relativposition der Cornea eine erste Relativposition eines Referenzkörpers mittels des nicht-interferometrischen Messsystems bestimmt, eine zweite Relativposition des Referenzkörpers wird mittels des optischen interferometrischen Messsystems bestimmt, und Abweichungen zwischen der ersten Relativposition des Referenzkörpers und der zweiten Relativposition der Referenzkörpers werden erfasst und beim Bestimmen der axialen Länge des Auges berücksichtigt.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 zeigt ein Blockdiagramm, welches schematisch eine ophthalmologische Messvorrichtung illustriert, welche ein interferometrisches Messsystem, ein nicht-interferometrisches Messsystem sowie Verarbeitungsmittel umfasst.
Figur 2 zeigt ein schematisches Querschnittsabbild eines Auges mit einem ersten Messbereich im Gebiet der Cornea und einem zweiten Messbereich im Gebiet der Retina.
Figur 3 zeigt ein Blockdiagramm, welches schematisch eine ophthalmologische Messvorrichtung illustriert, in der das interferometrische Messsystem eine Vorrichtung zur optischen Kohärenztomografie mit Tiefenabtastung umfasst.
Figur 4 zeigt eine grafische Darstellung eines Intensitätssignals im Zeitbereich (oben) und eines Spektrums im Frequenzbereich (unten), die aus der statischen interferometrischen Tiefenabtastung eines Auges resultieren.
Figur 5 zeigt ein Blockdiagramm, welches schematisch eine ophthalmologische Messvorrichtung illustriert, in der das interferometrische Messsystem als Michelson Interferometer mit veränderbarer Referenzarmlänge ausgeführt ist.
Figur 6 zeigt eine grafische Darstellung eines Intensitätssignals im Zeitbereich, das aus der interferometrischen Tiefenabtastung des Auges mittels veränderbarer Referenzarmlänge resultiert.
Figur 7 zeigt ein Blockdiagramm, welches schematisch eine ophthalmologische Messvorrichtung illustriert, in der das nicht-interferometrische Messsystem als Lichtschranke ausgeführt ist.
Figur 8 zeigt ein Blockdiagramm, welches schematisch eine ophthalmologische Messvorrichtung illustriert, in der das nicht-interferometrische Messsystem zur Detektierung der Cornea zwei sich in einem Kreuzungspunkt schneidende Lichtstrahlen projiziert.
Figur 9 zeigt ein Blockdiagramm, welches schematisch eine ophthalmologische Messvorrichtung illustriert, in der das nicht-interferometrische Messsystem eine optische Linse und eine Lochblende zur konfokalen Detektierung der Cornea umfasst.
Figuren 10a, 10b und 10c zeigen Blockdiagramme, welche jeweils schematisch ein nicht-interferometrisches Messsystem mit unterschiedlich angeordnetem Lichtprojektor und Lichtempfänger zur triangulierenden Bestimmung der Cornea illustrieren.
Figur 11 zeigt ein Blockdiagramm, welches schematisch eine ophthalmologische Messvorrichtung illustriert, in der das interferometrische Messsystem als Michelson Interferometer mit veränderbarer Referenzarmlänge und das nicht-interferometrische Messsystem mit in Scheimpflug angeordnetem Lichtempfänger und Lichtprojektor zur triangulierenden Bestimmung der Cornea ausgeführt sind.

### Wege zur Ausführung der Erfindung

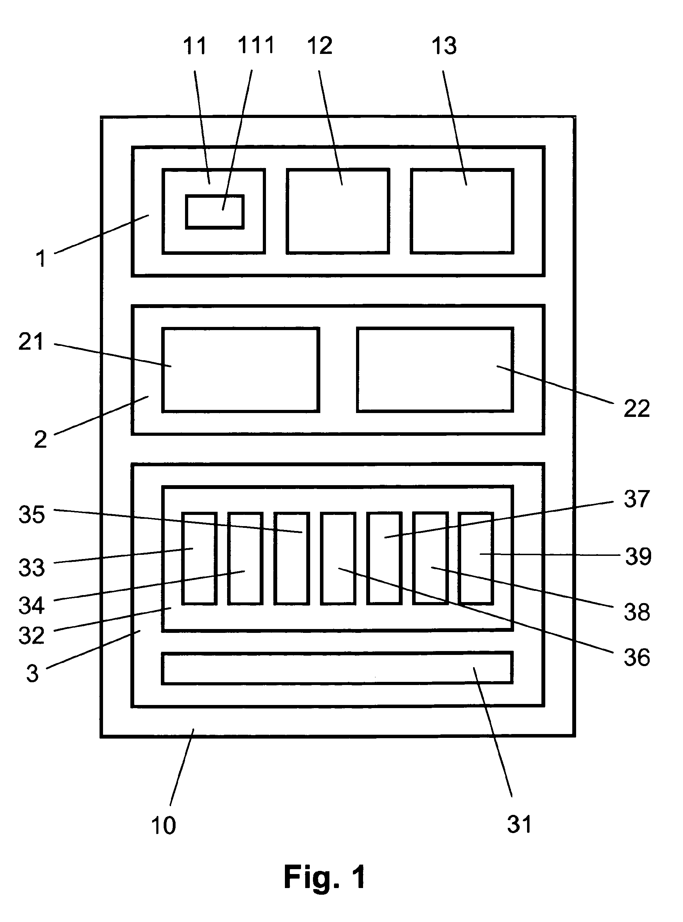

In den Figuren 1 bis 11 bezeichnen gleiche Bezugszeichen einander funktional entsprechende Komponenten und Systeme; die spezifischen Ausführungen dieser einander entsprechenden funktionalen Komponenten können jedoch in unterschiedlichen Ausführungsvarianten variieren.

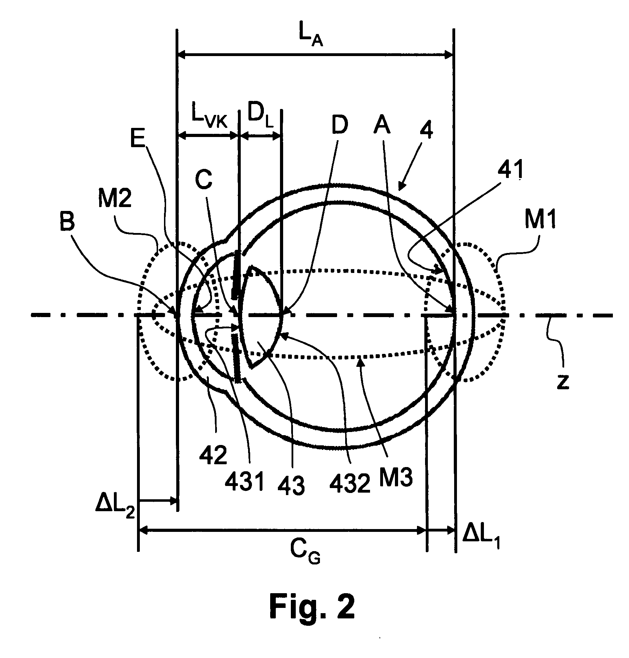

In der Figur 1 ist eine ophthalmologische Messvorrichtung 10 zum Bestimmen der axialen Länge L_{A} eines in der Figur 2 illustrierten Auges 4 dargestellt. Die ophthalmologische Messvorrichtung 10 umfasst ein interferometrisches Messsystem 1 zum Ermitteln einer relativen Position A der Retina 41 auf der Messachse z. Wie in der Figur 1 schematisch dargestellt ist umfasst das interferometrische Messsystem 1 eine kohärente bzw. teilkohärente Lichtquelle 111, strahlteilende und strahlvereinigende Mittel 12 (z.B. ein Strahlteilerwürfel), einen Lichtprojektor 11, einen Referenzarm 14 mit Spiegel, sowie einen Lichtdetektor 13. Die ophthalmologische Messvorrichtung 10 umfasst zudem ein nicht-interferometrisches Messsystem 2 zum Ermitteln einer relativen Position B der Cornea 42 auf der Messachse z. Die Messachse z entspricht vorzugsweise einer Längsachse des Auges 4, beispielsweise der optischen Achse oder der Sehachse. Die relative Position B der Cornea 42 auf der Messachse z entspricht dem Scheitelpunkt der Cornea 42. Wie in der Figur 1 schematisch dargestellt ist, umfasst das nicht-interferometrische Messsystem 2 einen Lichtprojektor 21 sowie einen Lichtempfänger 22. Die ophthalmologische Messvorrichtung 10 umfasst überdies Verarbeitungsmittel 3 mit einem Prozessor 31 und einem Programm- und Datenspeicher 32, in welchem verschiedene programmierte Softwaremodule zur Steuerung des Prozessors 31 gespeichert sind. Die Softwaremodule umfassen beispielsweise ein Berechnungsmodul 33, ein FFT-Modul 34, ein Triangulationsmodul 35, ein Spektroskopiemodul 36, ein Detektierungsmodul 37, ein Steuermodul 38 und/oder ein Bildverarbeitungsmodul 39. Das interferometrische Messsystem 1 ist vorzugsweise eingerichtet die relative Position A der Retina 41 innerhalb eines ersten Messbereichs M1 auf der Messachse z zu bestimmen. Das nicht-interferometrische Messsystem 2 ist vorzugsweise eingerichtet die relative Position B der Cornea 42 innerhalb eines zweiten Messbereichs M2 auf der Messachse z zu bestimmen. In einer bevorzugten Ausführungsvariante ist das interferometrische Messsystem 1 eingerichtet für Kalibrierungszwecke und für die Bestimmung (auf der Messachse z) der Relativposition E der Rückseite der Cornea 42, der Relativposition C der der Cornea 42 zugewandten Vorderseite 431 der Linse 43 sowie für die Bestimmung der Relativposition D der von Cornea 42 abgewandten Rückseite 432 der Linse 43 den erweiterten Messbereich M3 abzudecken (z.B. über eine Veränderung der Referenzarmlänge). Eine Veränderung der Referenzarmlänge kann zur Messbereichserweiterung von schnellen interferometrischen Verfahren mit begrenztem Messbereich genutzt werden. Die Verarbeitungsmittel 3 umfassen ein Berechnungsmodul 33, das eingerichtet ist aus der Relativposition A der Retina 41 und der Relativposition B der Cornea 42 die axiale Länge L_{A} des Auges 4 zu berechnen. Das Berechnungsmodul 33 ist zudem eingerichtet, die Vorderkammertiefe L_{VK} aus der Relativposition B der Cornea 42 und der Relativposition C der Vorderseite 431 der Linse 43 zu berechnen, die Dicke D_{L} der Linse 43 aus der Relativposition C der Vorderseite 431 der Linse 43 und der Relativposition D der Rückseite 432 der Linse 43 zu berechnen, und/oder die Dicke der Cornea 42 aus den Relativpositionen B und E der Vorderseite respektive Rückseite der Cornea 42 zu berechnen. Das Berechnungsmodul 33 ist zudem eingerichtet die berechneten Werte der axialen Länge L_{A}, der Vorderkammertiefe L_{VK}, der Hornhautdicke und/oder die Dicke D_{L} der Linse auf einer Anzeige der ophthalmologischen Messvorrichtung 10 anzuzeigen.

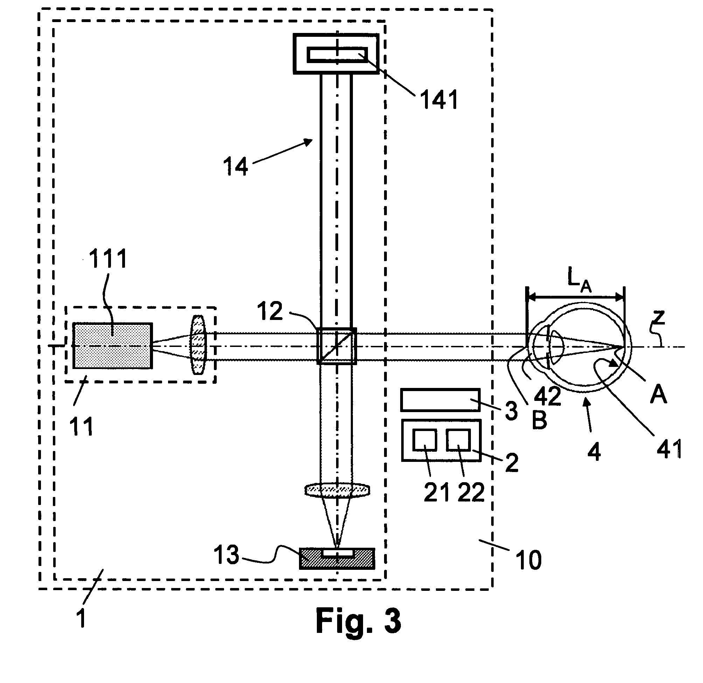

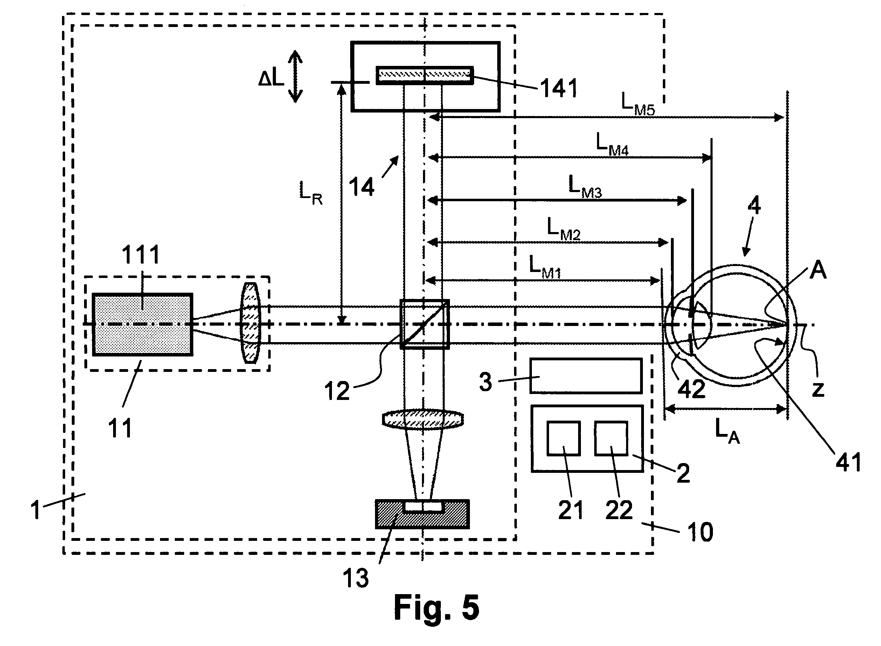

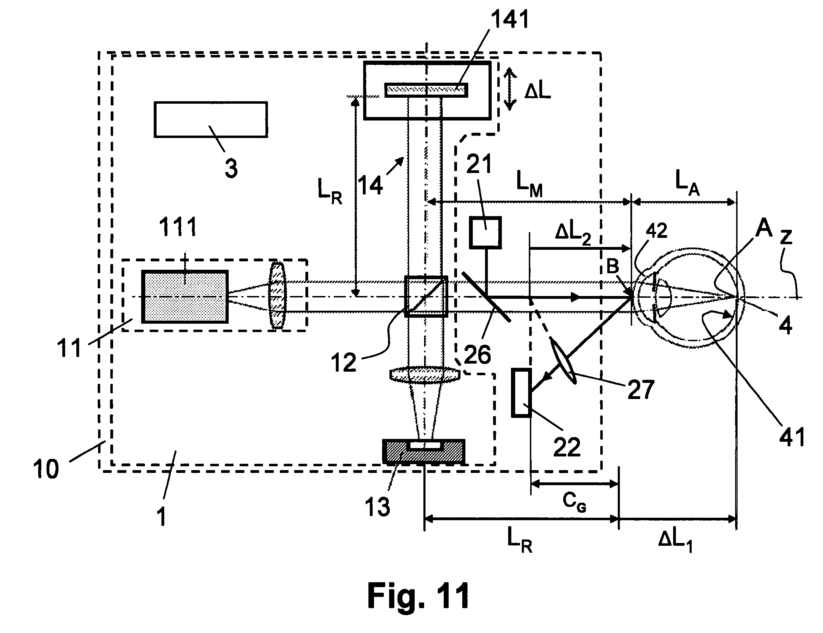

In den Figuren 3, 5 und 11 ist die ophthalmologische Messvorrichtung 10 jeweils mit einem interferometrischen Messsystem 1 dargestellt, das für die optische Kohärenztomografie (OCT) mit Tiefenabtastung eingerichtet ist. Das interferometrische Messsystem 1 respektive die Vorrichtung zur optischen Kohärenztomografie mit Tiefenabtastung umfasst einen Lichtprojektor 11 mit einer Lichtquelle 111, einen Strahlteiler 12, einen Referenzarm 14, sowie einen Lichtdetektor 13. Das interferometrische Messsystem 1 gemäss der Variante nach Figur 3 ist für die statische Tiefenabtastung (ohne veränderbare Referenzarmlänge) eingerichtet. In einer Ausführungsvariante ist das interferometrische Messsystem 1 nach Figur 3 als so genanntes Swept Source OCT (SSOCT) ausgeführt und umfasst eine Lichtquelle 111 mit veränderlicher Wellenlänge sowie einen Detektor 13 mit einer einfachen aber hochempfindlichen Photodiode. Das interferometrische Messsystem 1 nach Figur 3 kann auch als Frequency Domain OCT (FDOCT) oder Fourier (Transform) OCT (FOCT) mit breitbandiger Lichtquelle 111 und geeignetem Detektor 13 ausgeführt sein (z.B. Beugungsgitter mit CCD-Zeile). In der Ausführungsvariante nach den Figuren 5 und 11, ist das interferometrische Messsystem 1 als so genanntes Michelson Interferometer oder Time Domain OCT (TOCT) mit veränderbarer Referenzarmlänge (verschiebbarer Spiegel 141 im Referenzarm 14), breitbandiger Lichtquelle 111 und Detektor 13 mit einfacher aber hochempfindlicher Diode ausgeführt. Die Veränderung der Referenzarmlänge sowie unterschiedliche geeignete Methoden zur Signalverarbeitung werden z.B. in EP0581871 beschrieben.

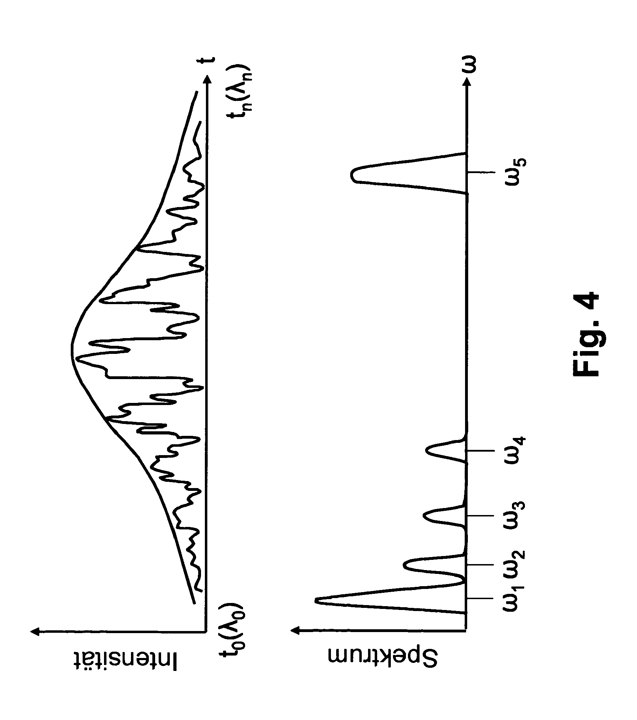

Die Figur 4 illustriert das vom Detektor 13 detektierte Intensitätssignal im Zeitbereich (oben) und das Spektrum im Frequenzbereich (unten) bei einer Tiefenabtastung des Auges 4 mittels des in der Figur 3 dargestellten interferometrischen Messsystems 1 in der SSOCT Ausführung. Das Intensitätssignal ist für die Wellenlänge λ₀ im Zeitpunkt t₀, bis zur Wellenlänge λₙ im Zeitpunkt tₙ dargestellt. Für die Bestimmung des Frequenzspektrums umfassen die Verarbeitungsmittel ein optionales FFT-Modul 34 (Fast Fourier Transformation). Im Frequenzspektrum werden die interferenzbildenden Grenzschichten im Auge 4 sichtbar, dabei entsprechen die Modulationsfrequenzen ω₁, ω₂, ω₃, ω₄, ω₅ den relativen Positionen (respektive Längen) der Grenzschichten und können für die Bestimmung der Relativposition A der Retina 41 (ω₅), der Relativposition C der Vorderseite 431 (ω₃) der Linse 43 sowie der Relativposition D der Rückseite 432 (ω₄) der Linse 43 verwendet werden. Insbesondere ermöglicht das Frequenzspektrum über die Basisposition ω₁ die relative Positionierung und/oder Kalibrierung der durch das interferometrische Messsystem 1 ermittelten Relativpositionen zu der durch das nicht-interferometrische Messsystem 2 bestimmten Relativposition B der Cornea respektive zur Geometrie der ophthalmologischen Messvorrichtung 10 (z.B. mittles eines Referenzkörpers, der von beiden Messsystemen erfasst werden kann).

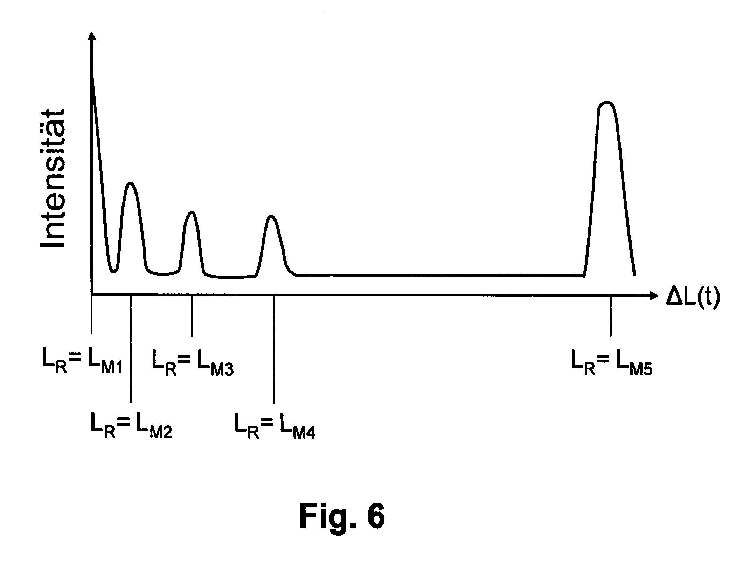

Die Figur 6 illustriert das vom Detektor 13 detektierte Intensitätssignal im Zeitbereich bei einer Tiefenabtastung des Auges 4 mittels veränderbarer Referenzarmlänge (z.B. über einen verschiebbaren Spiegel 141) des in den Figuren 5 und 11 dargestellten interferometrischen Messsystems 1 in TOCT Ausführung. Das Intensitätssignal ist abhängig von der Verschiebung ΔL (Abtastdistanz) des Referenzarms 14 dargestellt. Die interferenzbildenden Grenzschichten im Auge 4 sind im Zeitbereich als Funktion der Verschiebung sichtbar, dabei entsprechen die Messarmlängen L_{M1}, L_{M2}, L_{M3}, L_{M4} und L_{M5} den relativen Positionen (respektive Längen) der Grenzschichten und können für die Bestimmung der Relativposition A der Retina 41 (L_{M5}), der Relativposition C der Vorderseite 431 (L_{M3}) der Linse 43 sowie der Relativposition D der Rückseite 432 (L_{M4}) der Linse 43 verwendet werden. Insbesondere ermöglicht das Intensitätssignal über die Basisposition L_{M1} die relative Positionierung und/oder Kalibrierung der durch das interferometrische Messsystem 1 ermittelten Relativpositionen zu der durch das nicht-interferometrische Messsystem 2 bestimmten Relativposition B der Cornea respektive zur Geometrie der ophthalmologischen Messvorrichtung 10.

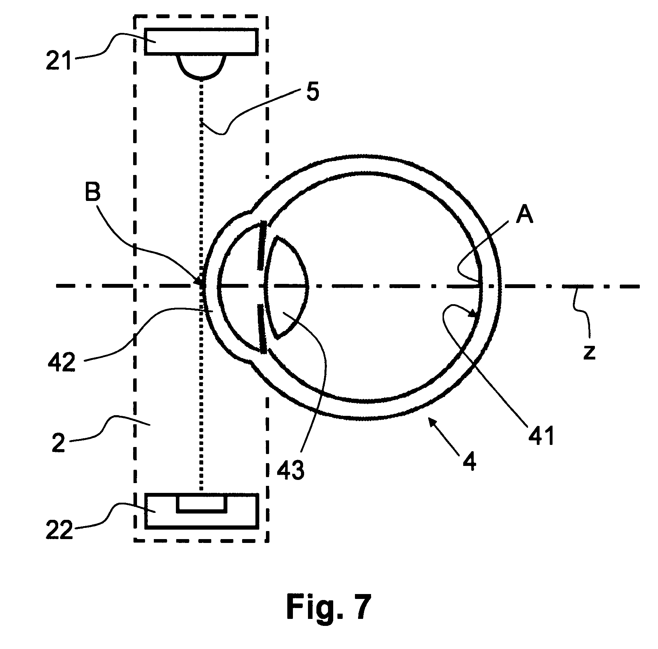

In einer bevorzugten Ausführungsvarianten ist das interferometrische Messsystem 1 nach einer der Figuren 3, 5 oder 11 direkt oder über ein optionales Steuermodul 38 so mit dem nicht-interferometrischen Messsystem 2 nach einer der Figuren 7, 8, 9, 10a, 10b, 10c oder 11 gekoppelt, dass ein Signal, das die interferometrische Detektierung der Retina 41 anzeigt, automatisch die nicht-interferometrische Bestimmung der Relativposition B der Cornea 42 durch das nicht-interferometrische Messsystem 2 aktiviert. Das die interferometrische Detektierung der Retina 41 anzeigende Signal wird beispielsweise automatisch durch ein optionales Detektierungsmodul 37 der Verarbeitungsmittel auf der Basis des Spektrumsverlaufs gemäss Figur 4 (unten) respektive des Intensitätssignals nach Figur 6 bestimmt. Das Berechnungsmodul 33 berechnet die axiale Länge L_{A} des Auges 4 automatisch aus der Relativposition A der Retina 41 und der Relativposition B der Cornea 42, sobald die durch das interferometrische Messsystem 1 getriggerte Bestimmung der Relativposition B der Cornea 42 durch das nicht-interferometrische Messsystem 2 vorliegt. In einer alternativen Ausführung berechnet das Berechnungsmodul 33 die axiale Länge L_{A} des Auges 4 laufend aus aktuellen Werten der Relativposition A der Retina 41 und der Relativposition B der Cornea 42.

In den Figuren 7, 8, 9 10a, 10b und 10c werden verschiedene Ausführungsformen für das nicht-interferometrische Messsystem 2 illustriert, die jeweils als unterschiedliche Ausführungsvarianten mit einer Variante des interferometrischen Messsystems 1, insbesondere mit den in den Figuren 3 und 5 illustrierten Ausführungsformen des interferometrischen Messsystems 1, kombinierbar sind. Es sei im Übrigen angemerkt, dass sich die Erfindung nicht nur auf Michelson Interferometer bezieht sondern prinzipiell mit allen Interferometern realisierbar ist, die eine relative Position bezüglich des Messgeräts liefern.

In der Ausführungsvariante nach Figur 7 sind der Lichtprojektor 21 und der Lichtempfänger 22 des nicht-interferometrischen Messsystems 2 so als Lichtschranke angeordnet, dass der Scheitelpunkt der Cornea 42 als Relativposition auf der Messachse z detektierbar ist. Das heisst die ophthalmologische Messvorrichtung 10 ist so positionierbar, dass das nicht-interferometrische Messsystem 2 den Messbereich M2 abdeckt und die genaue Lage der Cornea 42 in Bezug zur Messachse z durch Detektierung des Scheitelpunkts mittels der durch den Lichtprojektor 21, z.B. eine kollimierte Laserdiode, und den Lichtempfänger 22, z.B. ein Photosensor, gebildeten Lichtschranke ermitteln kann, wenn der Lichtstrahl 5 unterbrochen wird.

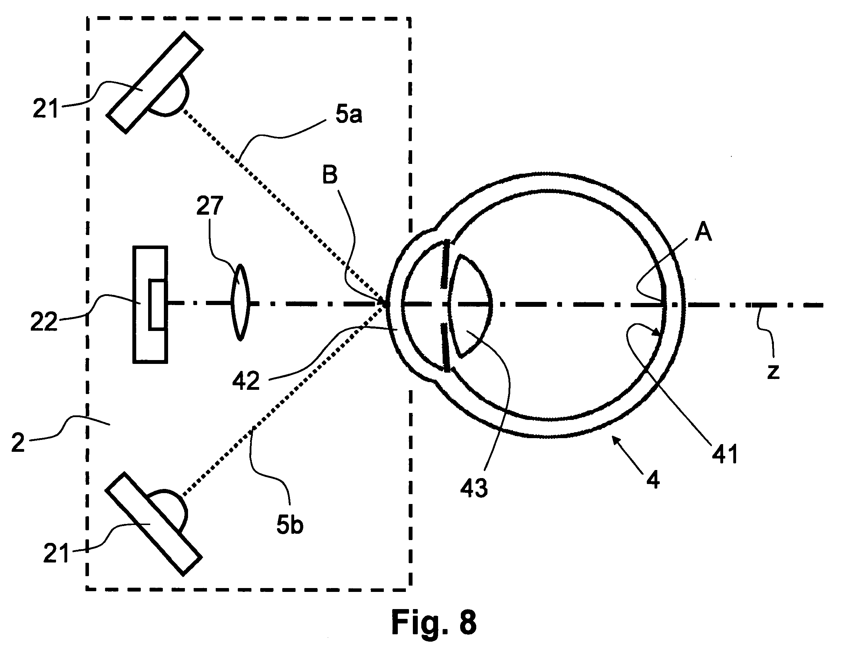

In der Ausführungsvariante nach Figur 8 ist das nicht-interferometrische Messsystem 2 eingerichtet zwei sich in einem Kreuzungspunkt schneidende Lichtstrahlen 5a, 5b zu projizieren, beispielsweise mittels zweier Lichtprojektoren 21 mit gemeinsamer Lichtquelle und entsprechend angeordneten Spiegeln. Die Lichtprojektoren 21, z.B. Laserdioden, und der Lichtempfänger 22, z.B. eine CCD-Kamera, sind so angeordnet, dass eine Positionierung des Kreuzungspunkts auf der Cornea 42 durch den Lichtempfänger 22 zur Bestimmung der Relativposition der Cornea 42 detektierbar ist. Das heisst der Messbereich M2 ist durch Positionieren des nicht-interferometrischen Messsystems 2 so abdeckbar, dass der Lichtempfänger 22 detektieren kann, wenn der Kreuzungspunkt der beiden Lichtstrahlen 5a, 5b auf Cornea 42, insbesondere auf den Scheitelpunkt der Cornea 42 zu liegen kommt, und die Lage der Cornea 42 in Bezug zur Messachse z ermittelt werden kann.

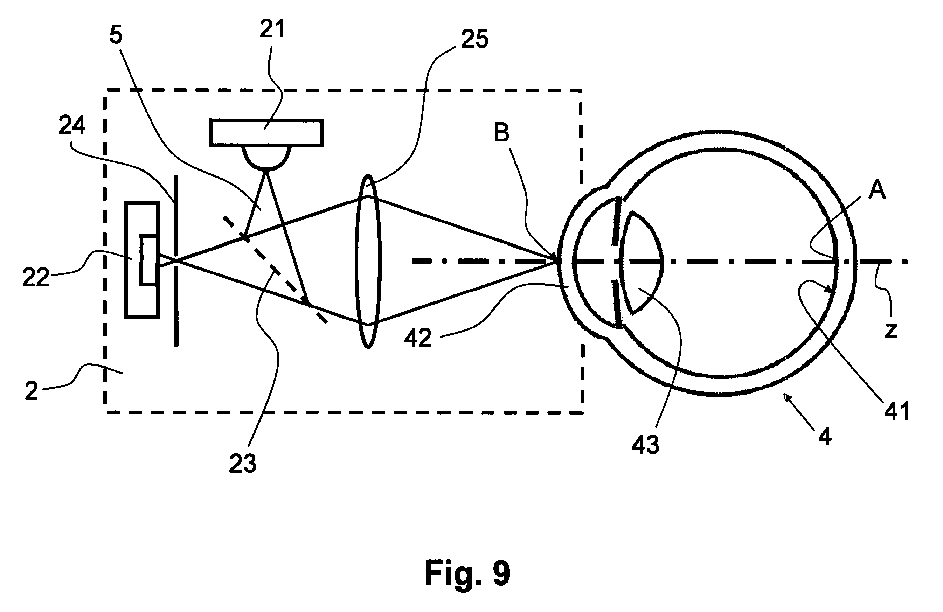

In der Ausführungsvariante nach Figur 9 umfasst das nicht-interferometrische Messsystem 2 zusätzlich zum Lichtprojektor 21 und zum Lichtempfänger 22 einen halbdurchlässigen Spiegel 23, eine Lochblende 24 und ein optisches System 25, z.B. eine optische Linse. Wie in der Figur 9 schematisch illustriert ist, wird der vom Lichtprojektor 21 abgegebene Lichtstrahl 5, z.B. ein Laserstrahl, über den Spiegel 23 und das optische System 25 fokussiert auf die Cornea 42 projiziert. Die Lochblende 24 ist beispielsweise so zwischen dem Lichtempfänger 22 und der optischen Linse 25 angeordnet, dass auf der Cornea 42 reflektiertes Licht dem Lichtempfänger, z.B. eine Photodiode, über das optische System 25 und die Lochblende 24 zugeführt wird, wenn die Cornea 42, insbesondere der Scheitelpunkt der Cornea 42, den gleichen fokalen Abstand von der optischen Linse 25 aufweist, wie die Lochblende 24. Das heisst der Messbereich M2 ist durch Positionieren des nicht-interferometrischen Messsystems 2 konfokal so abdeckbar, dass der Lichtempfänger 22 detektieren kann, wenn die Cornea 42, insbesondere der Scheitelpunkt der Cornea 42, in den Fokus der optischen Linse 25 zu liegen kommt und die Lage der Cornea 42 in Bezug zur Messachse z ermittelt werden kann. Das optische System 25 und die Lochblende 24 sind also so angeordnet, dass zur Bestimmung der Relativposition B der Cornea 42 das von der Cornea 42 reflektierte Licht durch das optische System 25 dem Lichtempfänger 22 zugeführt wird, wenn der Fokus des reflektierten Lichts in die Öffnung der Lochblende 24 zu liegen kommt.

In einer weiteren bevorzugten Ausführungsvarianten ist das nicht-interferometrische Messsystem 2 nach einer der Figuren 7, 8 oder 9 direkt oder über ein optionales Steuermodul 38 so mit dem interferometrischen Messsystem 1 gekoppelt, dass ein Signal, das die Lichtschrankendetektierung der Cornea 42 nach Figur 7, die Positionierung des Kreuzungspunkts auf der Cornea 42 nach Figur 8 oder die Detektierung der Cornea 42 im Fokuspunkt nach Figur 9 anzeigt, automatisch die interferometrische Bestimmung von wahlweise der Relativposition A der Retina, die Relativposition der Vorderseite C der Linse 43 und/oder die Relativposition der Rückseite D der Linse 43 durch das interferometrische Messsystem 1 aktiviert. Das Berechnungsmodul 33 berechnet die axiale Länge L_{A} des Auges 4 automatisch aus der Relativposition A der Retina 41 und der Relativposition B der Cornea 42, sobald die durch das nicht-interferometrische Messsystem 2 getriggerte Bestimmung der Relativposition A der Retina 41 durch das interferometrische Messsystem 1 vorliegt. In einer alternativen Ausführung berechnet das Berechnungsmodul 33 die axiale Länge L_{A} des Auges 4 laufend aus aktuellen Werten der Relativposition A der Retina 41 und der Relativposition B der Cornea 42.

In einer Variante ist das nicht-interferometrische Messsystem 2 nach einer der Figuren 7, 8 oder 9 als Autofokussystem für die automatische Messung der Distanz zur Cornea 42, insbesondere zum Scheitelpunkt der Cornea 42 ausgeführt. Zu diesem Zweck wird beispielsweise das gesamte Lichtschrankensystem nach Figur 7, 8 oder 9, die Lichtprojektoren nach Figur 8 oder die Lochblende 24 nach Figur 9 durch einen Bewegungstreiber automatisch bis zur Detektierung der Cornea 42 bewegt. Durch geeignete Kalibrierung können die Bewegungen in Relativpositionen umgewandelt werden.

In einer weiteren Variante umfasst das nicht-interferometrische Messsystem 2 zur Abstandsmessung einen Lichtprojektor mit starken chromatischen Aberrationen und eine breitbandige Lichtquelle wodurch sich eine wellenlängenabhängige Fokuslage ergibt. Die Verarbeitungsmittel 3 umfassen ein optionales Spektroskopiemodul 36, das eingerichtet ist, die Relativposition der Cornea 42, insbesondere des Scheitelpunkts der Cornea 42, auf der Basis der erfassten Spektren zu bestimmen, z.B. über entsprechende gespeicherte Tabellen.

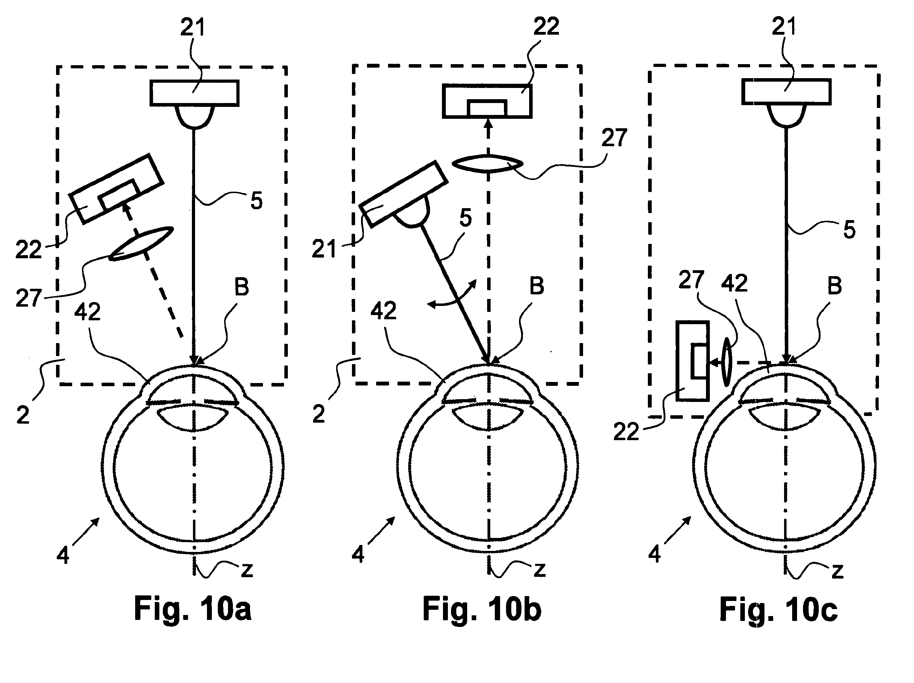

In den Figuren 10a, 10b und 10c sind verschiedene Anordnungen des Lichtprojektors 21 und des Lichtempfängers 22 des nicht-interferometrischen Messsystems 2 dargestellt, welche jeweils mittels Triangulation die Bestimmung der Relativposition B der Cornea 42, insbesondere des Scheitelpunkts der Cornea 42, basierend auf diffuser Reflexion auf Oberflächen respektive Streuung (bei Transparenz des Gewebes für die verwendete Wellenlänge) im Augengewebe ermöglichen.

In der Ausführungsvariante nach Figur 10a wird ein Lichtstrahl 5 oder (geformtes) Strahlenbündel, z.B. ein Laserstrahl, vom Lichtprojektor 21 entlang der Messachse z auf das Auge 4 gestrahlt. Der Lichtempfänger 22, beispielsweise ein einzeiliger CCD-Detektor mit abbildender Optik, ist ausserhalb der Strahlungsachse angeordnet und erfasst aus verschiedenen Positionen das Licht, das auf Oberflächen des Auges 4 diffus reflektiert und gegebenenfalls im Augengewebe gestreut wird. Ein optionales Triangulationsmodul 35 ist eingerichtet, aus den verschiedenen erfassten Perspektiven des reflektierten und gestreuten Lichts mittels Triangulationsverfahren die Relativposition B der Cornea 42, insbesondere des Scheitelpunkts der Cornea 42, zu bestimmen. In einer Untervariante ist der Lichtempfänger 22 beispielsweise eine CCD-Kamera, die in Scheimpfluganordnung zum Lichtstrahl respektive Strahlenbündel 5 angeordnet ist. Die Figur 10c zeigt eine Anordnung, in der die optische Achse des Lichtempfängers 22 senkrecht zur Projektionsachse angeordnet ist.

In der Ausführungsvariante nach Figur 11 umfasst die ophthalmologische Messvorrichtung 10 eine Kombination von Michelson Interferometer und nichtinterferometrischem Messsystem 2 in Scheimpfluganordnung. Das interferometrische Messsystem 1 ist als TOCT-System mit veränderlicher Referenzarmlänge (Referenzarm 14 mit verschiebbarem Spiegel 141), breitbandiger Lichtquelle 111 und Detektor 13 mit einfacher aber hochempfindlicher Photodiode ausgeführt. Im nicht-interferometrischen Messsystem 2 wird der Lichtstrahl des Lichtprojektors 21 über einen teildurchlässigen Spiegel 26 auf die Messachse z geleitet und das von der Cornea 42 diffus reflektierte respektive gestreute Licht wird über die optische Linse 27 auf den in Scheimpfluganordnung angebrachten Lichtempfänger 22, beispielsweise eine CCD-Kamera, gelenkt. Der Lichtempfänger 22 ist eingerichtet, ein Querschnittabbild des durch den Lichtprojektor 21 beleuchteten Querschnittteils in Scheimpfluganordnung zum Lichtstrahl zu erfassen und zu speichern. Das Triangulationsmodul 35 ist eingerichtet, die Relativposition B der Cornea 42 auf der Messachse z basierend auf dem gespeicherten Querschnittabbild zu bestimmen. Im Beispiel nach Figur 11 ergibt sich die axiale Länge L_{A} des Auges 4 aus L_{A} = C_{G} + ΔL₁ - ΔL₂, wobei ΔL₁ der Auslenkung des veränderbaren Referenzarms 14 entspricht, wobei ΔL₂ der durch das nicht-interferometrische Messsystem 2 ermittelten Relativposition B der Cornea 42, insbesondere des Scheitelpunkts der Cornea 42, entspricht, und wobei C_{G} einer geometrischen Systemkonstante der ophthalmologischen Messvorrichtung 10 entspricht, nämlich der Distanz der Referenznullpunkte der Messbereiche M1 und M2 des interferometrischen und des nicht-interferometrischen Messsystems 1, 2. Dies ist auch in Figur 2 ersichtlich, wo ΔL₁ dem Messwert vom Referenznullpunkt L_{R} des Messbereichs M1 des interferometrischen Messsystems 1 bis zur Relativposition A der Retina 41 entspricht; wo ΔL₂ dem Messwert vom Referenznullpunkt des Messbereichs M2 des nicht-interferometrischen Messsystems 2 bis zur Relativposition B der Cornea 42 entspricht; und wo die geometrische Systemkonstante C_{G} dem Abstand zwischen den Referenznullpunkten der Messbereiche M1 und M2 entspricht.

In der Ausführungsvariante nach Figur 10b ist der Lichtprojektor 21 eingerichtet den Lichtstrahl 5 oder das (geformte) Strahlenbündel zur Abtastung des Auges 4 respektive der Cornea 42 zu bewegen, so dass der Lichtempfänger 22, z.B. eine CCD-Kamera, diffus reflektiertes und gegebenenfalls im Augengewebe gestreutes Licht erfassen kann, dass in verschiedenen Projektionsrichtungen, z.B. in verschiedenen Projektionsebenen, auf das Auge 4 projiziert wird. Der Lichtempfänger 22 ist angeordnet das Licht entlang der Messachse z erfassen. Der Lichtprojektor 21 ist ausserhalb der Messachse z angeordnet. Das optionale Triangulationsmodul 35 ist eingerichtet, aus den erfassten Lichtreflexionen und gegebenenfalls Lichtstreuungen mittels Triangulationsverfahren die Relativposition B der Cornea 42, insbesondere des Scheitelpunkts der Cornea 42, zu bestimmen.

Vor der Verwendung der ophthalmologischen Messvorrichtung 10 zur Bestimmung der axialen Augenlänge L_{A}, der Vorderkammertiefe L_{VK}, der Hornhautdicke und/oder der Dicke D_{L} der Linse 43 erfolgt vorzugsweise eine Kalibrierung, beispielsweise basierend auf einem Referenzkörper bzw. Kalibrierkörper. Je nach Ausführungsvariante ist der Kalibrierkörper ein Referenzobjekt, das beispielsweise in der Form eines Kunstauges ausgeführt ist und das an der ophthalmologischen Messvorrichtung 10 angebracht und für Kalibrierungszwecke in den Messbereich M1, M2, M3 einschwenkbar ist, oder das nicht permanent sondern bloss für die Kalibrierung an der ophthalmologischen Messvorrichtung 10 entfernbar angebracht werden kann. In einer Ausführungsvariante ist der Kalibrierkörper als Kunstauge ausgeführt, das zwei Grenzflächen mit bekannten Abstand (bzw. optischer Weglänge) umfasst. Das Kunstauge kann z.B. eine gekrümmte Hornhaut aufweisen. Um die Detektierbarkeit zu erhöhen, kann zur Vermeidung von Interferenzringen der Kalibrierkörper auch plan ausgeführt werden. Die Hornhaut (d.h. vordere Grenzfläche) kann auch eine definierte Dicke aufweisen, um die Messung mit triangulierenden Verfahren zu ermöglichen. In einer weiteren Ausführungsvariante erfolgt die Kalibrierung über eine Grenzfläche, die durch beide Messsysteme 1, 2 gemessen werden kann. Das heisst die Messbereiche M1 und M2 überlappen sich, oder ein Messbereich kann vorübergehend und mit bekanntem Weg in den anderen verschoben werden. Zum Beispiel kann der Messbereich M2 des interferometrischen Messsystems 1 über einen beweglichen Referenzspiegel verschoben werden. Ist auch eine gekrümmte Hornhaut detektierbar, dann kann auch das menschliche Auge als Referenzkörper verwendet werden. Schliesslich kann die Kalibrierung auch über eine Grenzfläche ausgeführt werden, die über einen Bewegungstreiber definiert vom einen Messbereich in den anderen verschiebbar ist.

Eine erste Relativposition des Referenzkörpers wird mittels des nicht-interferometrischen Messsystems 2 bestimmt. Zudem wird mittels des optischen interferometrischen Messsystems 1 eine zweite Relativposition des Referenzkörpers bestimmt. Danach wird eine Abweichung zwischen der ersten Relativposition des Referenzkörpers und der zweiten Relativposition des Referenzkörpers ermittelt, gespeichert und beim Bestimmen der Augenabmessungen, insbesondere beim Bestimmen der axialen Länge L_{A} des Auges 4, berücksichtigt.

Für den Messvorgang zur berührungsfreien Bestimmung der axialen Augenlänge L_{A} bieten sich je nach Ausführungsvariante der ophthalmologischen Messvorrichtung 10 die folgenden Varianten an:
a) Kontinuierliche und laufende Bestimmung der Relativposition A der Retina 41 mittels des optischen interferometrischen Messsystems 1 und der Relativposition B der Cornea 42 mittels des nicht-interferometrischen Messsystems 2, und laufende Berechnung der axialen Augenlänge L_{A} auf der Basis der aktuellen Werte der Relativposition A der Retina 41 und der Relativposition B der Cornea 42. Der Wert der axialen Augenlänge L_{A} wird beispielsweise erst dann angezeigt, wenn sich der Wert innerhalb eines definierten Schwankungsbereichs eingepegelt hat.
b) Positionieren der ophthalmologischen Messvorrichtung 10 für die Erfassung des Messbereichs M2 durch das nicht-interferometrische Messsystem 2, und Bestimmen und Speichern der Relativposition B der Cornea 42 mittels des nicht-interferometrischen Messsystems 2. Automatische Aktivierung des interferometrischen Messsystems 1 zur Bestimmung und Speicherung der Relativposition A der Retina 41, wenn die Relativposition B der Cornea 42 durch das nicht-interferometrische Messsystem 2 detektiert wird. Berechnung der axialen Augenlänge L_{A} basierend auf den (temporär) gespeicherten Werten der Relativposition A der Retina 41 und der Relativposition B der Cornea 42.
c) Positionieren der ophthalmologischen Messvorrichtung 10 für die Erfassung des Messbereichs M1 durch das interferometrische Messsystem 1, und Bestimmen und Speichern der Relativposition A der Retina 41 mittels des interferometrischen Messsystems 1. Automatische Aktivierung des nicht-interferometrischen Messsystems 2 zur Bestimmung und Speicherung der Relativposition B der Cornea 42, wenn die Relativposition A der Retina 41 durch das interferometrische Messsystem 1 detektiert wird. Berechnung der axialen Augenlänge L_{A} basierend auf den gespeicherten Werten der Relativposition A der Retina 41 und der Relativposition B der Cornea 42.

Es sei noch angemerkt, dass je nach Ausführung des nicht interferometrischen Systems von diesem die Hornhautdicke, die Vorderkammertiefe und die Linsendicke gemessen werden kann.

## Patentansprüche

1. Messverfahren zur berührungsfreien Bestimmung der axialen Länge (LA) eines Auges (4), umfassend:
Bestimmung einer Relativposition (A) der Retina (41) des Auges (4) mittels eines optischen interferometrischen Messsystems (1), und
Bestimmen einer Relativposition (B) der Cornea (42) des Auges mittels eines nicht-interferometrischen Messsystem (2),
**dadurch gekennzeichnet,**
**dass** vor der Bestimmung der Relativposition (A) der Retina (41) und der Relativposition (B) der Cornea (42) eine erste Relativposition eines Referenzkörpers mittels des nicht-interferometrischen Messsystems (2) bestimmt wird,
**dass** eine zweite Relativposition des Referenzkörpers mittels des optischen interferometrischen Messsystems (1) bestimmt wird,
**dass** eine Abweichung zwischen der ersten Relativposition des Referenzkörpers und der zweiten Relativposition des Referenzkörpers erfasst wird, und
**dass** die axiale Länge (LA) des Auges (4) basierend auf der Relativposition (A) der Retina (41) und der Relativposition (B) der Cornea (42) und unter Berücksichtigung der erfassten Abweichung zwischen der ersten Relativposition des Referenzkörpers und der zweiten Relativposition des Referenzkörpers bestimmt wird.

2. Messverfahren nach Anspruch 1, **dadurch gekennzeichnet, dass** mittels eines Lichtschrankensystems ein Scheitelpunkt der Cornea (42) als Relativposition (B) der Cornea (42) detektiert wird, und dass bei einer Detektierung des Scheitelpunkts automatisch das optische interferometrische Messsystem (1) zur Bestimmung der Relativposition (A) der Retina (41) aktiviert wird.

3. Ophthalmologische Messvorrichtung (10) zum Bestimmen der axialen Länge (LA) eines Auges (4), umfassend:
ein optisches interferometrisches erstes Messsystem (1) zur Bestimmung einer Relativposition (A) der Retina (41) des Auges (4), und
ein nicht-interferometrisches zweites Messsystem (2) zur Bestimmung einer Relativposition (B) der Cornea (42) des Auges (4), **gekennzeichnet durch** Verarbeitungsmittel (3) zum Bestimmen der axialen Länge (LA) des Auges (4) basierend auf der Relativposition (A) der Retina (41) und der Relativposition (B) der Cornea (42) und unter Berücksichtigung einer gespeicherten Abweichung zwischen einer mittels des nicht-interferometrischen Messsystems (2) vor der Bestimmung der Relativposition (A) der Retina (41) und der Relativposition (B) der Cornea (42) bestimmten ersten Relativposition eines Referenzkörpers und einer mittels des optischen interferometrischen Messsystems (1) bestimmten zweiten Relativposition des Referenzkörpers.

4. Messvorrichtung (10) nach Anspruch 3, **dadurch gekennzeichnet, dass** das zweite Messsystem (2) ein Abstandsmesssystem umfasst zur Bestimmung der Relativposition (B) der Cornea (42) auf der Basis von vom Auge (4) diffus reflektiertem und/oder gestreutem Licht.

5. Messvorrichtung (10) nach Anspruch 3, **dadurch gekennzeichnet, dass** das zweite Messsystem (2) ein Abstandsmesssystem umfasst zur Bestimmung der Relativposition (B) der Cornea (42) auf der Basis von von der Cornea (42) spiegelnd reflektiertem Licht.

6. Messvorrichtung (10) nach einem der Ansprüche 3 bis 5, **dadurch gekennzeichnet, dass** das zweite Messsystem (2) einen Lichtprojektor (21) zur Projektion mindestens eines Lichtstrahls auf die Cornea (42) umfasst, dass das zweite Messsystem (2) einen mit den Verarbeitungsmitteln (3) verbundenen Lichtempfänger (22) umfasst, und dass die Verarbeitungsmittel (3) eingerichtet sind, auf Grund eines vom Lichtempfänger (22) empfangenen Signals die Relativposition (B) der Cornea (42) zu bestimmen.

7. Messvorrichtung (10) nach Anspruch 6, **dadurch gekennzeichnet, dass** der Lichtprojektor (21) eingerichtet ist, zwei sich in einem Kreuzungspunkt schneidende Lichtstrahlen zu projizieren, und dass der Lichtempfänger (22) eingerichtet ist, zur Bestimmung der Relativposition (B) der Cornea (42) eine Positionierung des Kreuzungspunkts auf der Cornea (42) zu detektieren.

8. Messvorrichtung (10) nach Anspruch 6, **dadurch gekennzeichnet, dass** das zweite Messsystem (2) ein optisches System (25) zur fokussierten Projektion des Lichtstrahls auf die Cornea (42) umfasst, dass das zweite Messsystem (2) eine dem Lichtempfänger (22) vorgeschaltete Lochblende (24) umfasst, wobei das optische System (25) und die Lochblende (24) so angeordnet sind, dass zur Bestimmung der Relativposition (B) der Cornea (42) der von der Cornea (42) reflektierte Lichtstrahl über das optische System (25) und durch die Lochblende (24) dem Lichtempfänger (22) zugeführt wird.

9. Messvorrichtung (10) nach einem der Ansprüche 5 bis 8, **dadurch gekennzeichnet, dass** das Abstandsmesssystem einen Autofokussensor zur Bestimmung der Relativposition (B) der Cornea (42) umfasst.

10. Messvorrichtung (10) nach Anspruch 6, **dadurch gekennzeichnet, dass** das Abstandsmesssystem einen Sensor umfasst zur Detektierung von wellenlängenabhängigen Brennpunkten bei Fokussierung einer breitbandigen Lichtquelle mit einer Optik mit starken chromatischen Aberrationen, und dass die Verarbeitungsmittel (3) eingerichtet sind auf Grund der chromatischen Aberrationen die Relativposition (B) der Cornea (42) zu bestimmen.

11. Messvorrichtung (10) nach einem der Ansprüche 3, 6, 7 oder 8, **dadurch gekennzeichnet, dass** das zweite Messsystem (2) ein Lichtschrankensystem zur Detektierung eines Scheitelpunkts der Cornea (42) als Relativposition (B) der Cornea (42) umfasst, und dass das Lichtschrankensystem so mit dem ersten Messsystem (1) gekoppelt ist, dass die Detektierung des Scheitelpunkts automatisch das erste Messsystem (1) zur Bestimmung der Relativposition (A) der Retina (41) aktiviert.

12. Messvorrichtung (10) nach einem der Ansprüche 3 bis 11, **dadurch gekennzeichnet, dass** das erste Messsystem (1) zudem eingerichtet ist zur Bestimmung einer Relativposition (C) einer der Cornea (42) zugewandten Vorderseite (431) der Linse (43) des Auges (4), und dass die Verarbeitungsmittel (3) zudem eingerichtet sind, basierend auf der Relativposition (B) der Cornea (42) und der Relativposition (C) der zugewandten Vorderseite (431) die Vorderkammertiefe (LVK) des Auges (4) zu bestimmen.

13. Messvorrichtung (10) nach einem der Ansprüche 3 bis 12, **dadurch gekennzeichnet, dass** das erste Messsystem (1) zudem eingerichtet ist zur Bestimmung einer Relativposition (D) einer von der Cornea (42) abgewandten Rückseite (432) der Linse (43), und dass die Verarbeitungsmittel (3) zudem eingerichtet sind, basierend auf einer Relativposition (C) einer der Cornea (42) zugewandten Vorderseite (431) der Linse (43) des Auges (4) und der Relativposition (D) der abgewandten Rückseite (432) eine Dicke (DL) der Linse (43) zu bestimmen.

14. Messvorrichtung (10) nach einem der Ansprüche 3 bis 13, **dadurch gekennzeichnet, dass** das erste Messsystem (1) und das zweite Messsystem (2) jeweils eine Lichtquelle zur Erzeugung eines Lichtstrahls umfassen, wobei die Wellenlängen der von den Lichtquellen erzeugten Lichtstrahlen voneinander verschieden sind.

## Claims

1. Measurement method for contactlessly determining the axial length (LA) of an eye (4), comprising:
determining a relative position (A) of the retina (41) of the eye (4) by means of an optical interferometric measurement system (1), and
determining a relative position (B) of the cornea (42) of the eye by means of a non-interferometric measurement system (2),
**characterized**
**in that** before the determination of the relative position (A) of the retina (41) and of the relative position (B) of the cornea (42), a first relative position of a reference body is determined by means of the non-interferometric measurement system (2),
**in that** a second relative position of the reference body is determined by means of the optical interferometric measurement system (1),
**in that** a deviation between the first relative position of the reference body and the second relative position of the reference body is acquired, and
**in that** the axial length (LA) of the eye (4) is determined on the basis of the relative position (A) of the retina (41) and of the relative position (B) of the cornea (42) and by taking into account the acquired deviation between the first relative position of the reference body and the second relative position of the reference body.

2. Measurement method according to Claim 1, **characterized in that** a vertex of the cornea (42) is detected as relative position (B) of the cornea (42) by means of a light barrier system, and **in that** upon the detection of the vertex the optical interferometric measurement system (1) is automatically activated in order to determine the relative position (A) of the retina (41).

3. Ophthalmological measurement apparatus (10) for determining the axial length (LA) of an eye (4), comprising:
an optical interferometric first measurement system (1) for determining a relative position (A) of the retina (41) of the eye (4), and
a non-interferometric second measurement system (2) for determining a relative position (B) of the cornea (42) of the eye (4), **characterized by**
processing means (3) for determining the axial length (LA) of the eye (4) on the basis of the relative position (A) of the retina (41) and of the relative position (B) of the cornea (42) and by taking into account a stored deviation between a first relative position of a reference body, determined by means of the non-interferometric measurement system (2) before the determination of the relative position (A) of the retina (41) and the relative position (B) of the cornea (42), and a second relative position of the reference body, determined by means of the optical interferometric measurement system (1).

4. Measurement apparatus (10) according to Claim 3, **characterized in that** the second measurement system (2) comprises a distance measurement system for determining the relative position (B) of the cornea (42) on the basis of light diffusely reflected and/or scattered by the eye (4).

5. Measurement apparatus (10) according to Claim 3, **characterized in that** the second measurement system (2) comprises a distance measurement system for determining the relative position (B) of the cornea (42) on the basis of light specularly reflected by the cornea (42).

6. Measurement apparatus (10) according to one of Claims 3 to 5, **characterized in that** the second measurement system (2) comprises a light projector (21) for projecting at least one light beam onto the cornea (42), **in that** the second measurement system (2) comprises a light receiver (22) connected to the processing means (3), and **in that** the processing means (3) are set up to determine the relative position (B) of the cornea (42) on the basis of a signal received by the light receiver (22).

7. Measurement apparatus (10) according to Claim 6, **characterized in that** the light projector (21) is set up to project two light beams intersecting at a crossing point, and **in that** the light receiver (22) is set up to detect a positioning of the crossing point on the cornea (42) in order to determine the relative position (B) of the cornea (42).

8. Measurement apparatus (10) according to Claim 6, **characterized in that** the second measurement system (2) comprises an optical system (25) for focussed projection of the light beam onto the cornea (42), **in that** the second measurement system (2) comprises a pinhole diaphragm (24) placed upstream of the light receiver (22), the optical system (25) and the pinhole diaphragm (24) being arranged such that the light beam reflected by the cornea (42) is fed to the light receiver (22) via the optical system (25) and through the pinhole diaphragm (24) in order to determine the relative position (B) of the cornea (42).

9. Measurement apparatus (10) according to one of Claims 5 to 8, **characterized in that** the distance measurement system comprises an autofocus sensor for determining the relative position (B) of the cornea (42).

10. Measurement apparatus (10) according to Claim 6, **characterized in that** the distance measurement system comprises a sensor for detecting wavelength-dependent focal points when focussing a broadband light source with the aid of an optical system having strong chromatic aberrations, and **in that** the processing means (3) are set up to determine the relative position (B) of the cornea (42) on the basis of the chromatic aberrations.

11. Measurement apparatus (10) according to one of Claims 3, 6, 7 or 8, **characterized in that** the second measurement system (2) comprises a light barrier system for detecting a vertex of the cornea (42) as relative position (B) of the cornea (42), and **in that** the light barrier system is coupled to the first measurement system (1) such that the detection of the vertex automatically activates the first measurement system (1) in order to determine the relative position (A) of the retina (41).

12. Measurement apparatus (10) according to one of Claims 3 to 11, **characterized in that** the first measurement system (1) is additionally set up to determine a relative position (C) of a front side (431), facing the cornea (42), of the lens (43) of the eye (4), and **in that** the processing means (3) are additionally set up to determine the anterior chamber depth (LVK) of the eye (4) on the basis of the relative position (B) of the cornea (42) and of the relative position (C) of the facing front side (431).

13. Measurement apparatus (10) according to one of Claims 3 to 12, **characterized in that** the first measurement system (1) is additionally set up to determine a relative position (D) of a rear side (432), averted from the cornea (42), of the lens (43), and **in that** the processing means (3) are additionally set up to determine a thickness (DL) of the lens (43) on the basis of a relative position (C) of a front side (431), facing the cornea (42), of the lens (43) of the eye (4) and of the relative position (D) of the averted rear side (432).

14. Measurement apparatus (10) according to one of Claims 3 to 13, **characterized in that** the first measurement system (1) and the second measurement system (2) each comprise a light source for generating a light beam, the wavelengths of the light beams generated by the light sources being different from one another.

## Revendications

1. Procédé de mesure pour la détermination sans contact de la longueur axiale (LA) d'un oeil (4), comprenant :
la détermination d'une position relative (A) de la rétine (41) de l'oeil (4) au moyen d'un système de mesure optique interférométrique (1), et
la détermination d'une position relative (B) de la cornée (42) de l'oeil au moyen d'un système de mesure non interférométrique (2),
**caractérisé**
**en ce qu'**avant la détermination de la position relative (A) de la rétine (41) et de la position relative (B) de la cornée (42), on détermine une première position relative d'un corps de référence au moyen du système de mesure non interférométrique (2),
**en ce qu'**on détermine une deuxième position relative du corps de référence au moyen du système de mesure optique interférométrique (1),
**en ce qu'**on enregistre un écart entre la première position relative du corps de référence et la deuxième position relative du corps de référence, et
**en ce qu'**on détermine la longueur axiale (LA) de l'oeil (4) sur la base de la position relative (A) de la rétine (41) et de la position relative (B) de la cornée (42) et en tenant compte de l'écart enregistré entre la première position relative du corps de référence et la deuxième position relative du corps de référence.

2. Procédé de mesure selon la revendication 1, **caractérisé en ce qu'**au moyen d'un système de barrière lumineuse, on détecte un sommet de la cornée (42) comme position relative (B) de la cornée (42) et **en ce que** lors d'une détection du sommet, le système de mesure optique interférométrique (1) est activé automatiquement pour la détermination de la position relative (A) de la rétine (41).

3. Dispositif de mesure ophtalmologique (10) pour déterminer la longueur axiale (LA) d'un oeil (4), comprenant:
un premier système de mesure optique interférométrique (1) pour la détermination d'une position relative (A) de la rétine (41) de l'oeil (4), et
un deuxième système de mesure non interférométrique (2) pour la détermination d'une position relative (B) de la cornée (42) de l'oeil (4),
**caractérisé par**
des moyens de traitements (3) pour déterminer la longueur axiale (LA) de l'oeil (4) sur la base de la position relative (A) de la rétine (41) et de la position relative (B) de la cornée (42) et en tenant compte d'un écart enregistré entre une première position relative d'un corps de référence déterminée au moyen du système de mesure non interférométrique (2) avant la détermination de la position relative (A) de la rétine (41) et de la position relative (B) de la cornée (42), et une deuxième position relative du corps de référence déterminée au moyen du système de mesure optique interférométrique (1).

4. Dispositif de mesure (10) selon la revendication 3, **caractérisé en ce que** le deuxième système de mesure (2) comprend un système de mesure de distance pour la détermination de la position relative (B) de la cornée (42) sur la base de la lumière réfléchie de manière diffuse et/ou diffusée par l'oeil (4).

5. Dispositif de mesure (10) selon la revendication 3, **caractérisé en ce que** le deuxième système de mesure (2) comprend un système de mesure de distance pour la détermination de la position relative (B) de la cornée (42) sur la base de la lumière réfléchie de manière spéculaire par la cornée (42).

6. Dispositif de mesure (10) selon l'une des revendications 3 à 5, **caractérisé en ce que** le deuxième système de mesure (2) comprend un projecteur de lumière (21) pour la projection d'au moins un rayon lumineux sur la cornée (42), **en ce que** le deuxième système de mesure (2) comprend un récepteur de lumière (22) relié aux moyens de traitements (3), et **en ce que** les moyens de traitements (3) sont conçus pour déterminer la position relative (B) de la cornée (42) sur la base d'un signal reçu par le récepteur de lumière (22).

7. Dispositif de mesure (10) selon la revendication 6, **caractérisé en ce que** le projecteur de lumière (21) est conçu pour projeter deux rayons lumineux se coupant en un point de croisement, et **en ce que** le récepteur de lumière (22) est conçu pour détecter un positionnement du point de croisement sur la cornée (42) pour la détermination de la position relative (B) de la cornée (42).

8. Dispositif de mesure (10) selon la revendication 6, **caractérisé en ce que** le deuxième système de mesure (2) comprend un système optique (25) pour la projection focalisée du rayon lumineux sur la cornée (42), **en ce que** le deuxième système de mesure (2) comprend un diaphragme à trou (24) placé devant le récepteur de lumière (22), le système optique (25) et le diaphragme à trou (24) étant disposés de telle sorte que pour la détermination de la position relative (B) de la cornée (42), le rayon lumineux réfléchi par la cornée (42) est acheminé vers le récepteur de lumière (22) par l'intermédiaire du système optique (25) et à travers le diaphragme à trou (24).

9. Dispositif de mesure (10) selon l'une des revendications 5 à 8, **caractérisé en ce que** le système de mesure de distance comprend un capteur d'autofocus pour la détermination de la position relative (B) de la cornée (42).

10. Dispositif de mesure (10) selon la revendication 6, **caractérisé en ce que** le système de mesure de distance comprend un capteur pour la détection de points focaux dépendant de la longueur d'ondes en focalisant une source lumineuse à large bande au moyen d'une optique présentant de fortes aberrations chromatiques, et **en ce que** les moyens de traitements (3) sont conçus pour déterminer la position relative (B) de la cornée (42) sur la base des aberrations chromatiques.

11. Dispositif de mesure (10) selon l'une des revendications 3, 6, 7 ou 8, **caractérisé en ce que** le deuxième système de mesure (2) comprend un système de barrière lumineuse pour détecter un sommet de la cornée (42) comme position relative (B) de la cornée (42) et **en ce que** le système de barrière lumineuse est couplé au premier système de mesure (1) de telle sorte que la détection du sommet active automatiquement le premier système de mesure (1) pour la détermination de la position relative (A) de la rétine (41).

12. Dispositif de mesure (10) selon l'une des revendications 3 à 11, **caractérisé en ce que** le premier système de mesure (1) est de plus conçu pour la détermination d'une position relative (C) d'une face avant (431) du cristallin (43) de l'oeil (4) orientée vers la cornée (42), et **en ce que** les moyens de traitements (3) sont de plus conçus pour déterminer la profondeur de la chambre antérieure (LVK) de l'oeil (4) sur la base de la position relative (B) de la cornée (42) et de la position relative (C) de la face avant (431) orientée vers elle.

13. Dispositif de mesure (10) selon l'une des revendications 3 à 12, **caractérisé en ce que** le premier système de mesure (1) est de plus conçu pour la détermination d'une position relative (D) d'une face arrière (432) du cristallin (43) éloignée de la cornée (42), et **en ce que** les moyens de traitements (3) sont de plus conçus pour déterminer une épaisseur (DL) du cristallin (43) sur la base d'une position relative (C) d'une face avant (431) du cristallin (43) de l'oeil (4) orientée vers la cornée (42) et de la position relative (D) de la face arrière (432) opposée.

14. Dispositif de mesure (10) selon l'une des revendications 3 à 13, **caractérisé en ce que** le premier système de mesure (1) et le deuxième système de mesure (2) comportent chacun une source lumineuse pour produire un rayon lumineux, les longueurs d'ondes des rayons lumineux produits par les sources lumineuses étant différentes les unes des autres.
